# EUROPEAN PATENT APPLICATION

(11) **EP 2 191 858 A1**
(43) Date of publication of application: **02.06.2010**
(21) Application number: 08828444.3
(22) Date of filing: 21.08.2008
(51) Int. Cl.: A61M 1/36

(54) **BLOOD STORAGE VESSEL**

(30) Priority: 27.08.2007 JP 2007220211
(71) Applicant: JMS Co., Ltd., Hiroshima-shi Hiroshima 730-8652 (JP)
(72) Inventor: KATSUNO, Yutaka, Hiroshima 730-8652 (JP); TANAKA, Minoru, Hiroshima 730-8652 (JP)
(74) Representative: Diehl & Partner
(86) International application number: PCT/JP2008/064922
(87) International publication number: WO 2009/028395

(57) **Abstract**

A cardiotomy section (2) includes a filter (10) and a defoamer (20) arranged inside the filter. A lower end (11a) of the filter is sealed or folded in an approximately straight line or in a curve, and the defoamer is annularly arranged along the inner peripheral surface of the filter, as viewed from above. A conduit tube (90) that introduces intracardiac blood is inserted from above downward in the cardiotomy section. A lower end of the conduit tube is located at the same level as or at a lower level than the lower end of the defoamer, with respect to the vertical direction. In a projection view of a lower end face of the defoamer with respect to a horizontal plane, (Ru2-2×Tu)/(Ru1-2×Tu)≤0.24 is satisfied, where Ru1 is the outer dimension of the annularly arranged defoamer in a major axial direction, Ru2 is the outer dimension thereof in a minor axial direction, and Tu is the thickness of the defoamer. This achieves a blood reservoir equipped with a cardiotomy section having high defoaming performance.

## Description

### Technical Field

The present invention relates to a blood reservoir that temporarily stores extracorporeally circulating blood in an extracorporeal circulation circuit for use in cardiopulmonary surgeries or the like. In particular, it relates to a blood reservoir equipped with a built-in cardiotomy section that filters intracardiac blood.

### Background Art

For cardiac surgeries or the like, an extracorporeal circulation circuit equipped with a blood pump or an artificial lung serving as a substitute for the function of a patient's heart or lung is used. Such an extracorporeal circulation circuit is provided with a blood reservoir (sometimes referred to as a "venous blood reservoir") for temporarily storing venous blood removed from a patient's vein and adjusting the blood volume in a circulating circuit, and a blood reservoir (sometimes referred to as an "cardiotomy reservoir") for aspirating, collecting, and temporarily storing blood (intracardiac blood) flowing out of the operative field. As compared to the venous blood, the intracardiac blood contains a high proportion of air bubbles or extraneous materials, such as pieces of flesh, fats, and clots, so the cardiotomy reservoir is provided with a cardiotomy section composed of a filter for removing extraneous materials and a defoamer for defoaming. Storing both of the venous blood and the intracardiac blood in a common blood reservoir is also widely practiced.

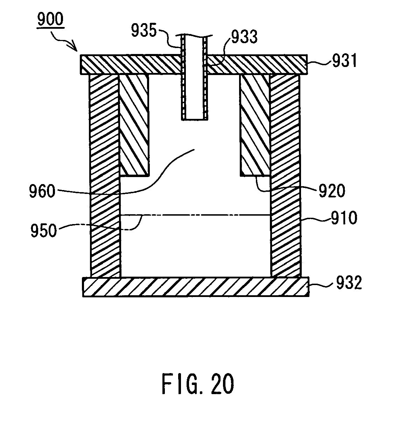

FIG. 20 is a cross-sectional view illustrating an example of a general configuration of a conventional cardiotomy section 900. The cardiotomy section 900 includes a filter 910 having a generally cylindrical shape as a whole and a defoamer 920 having a generally cylindrical shape and located inside the filter 910. Generally disk-shaped resin plates 931 and 932 are bonded to the upper and lower edges of the filter 910. The defoamer 920 is held by being bonded to the upper resin plate 931. The upper resin plate 931 has a through hole 933 formed in the center thereof. A conduit tube 935 that introduces intracardiac blood into the cardiotomy section 900 is inserted in the through hole 933. A chain double-dashed line 950 indicates a maximum blood surface level. The defoamer 920 is located at a higher level than the maximum blood surface level 950.

The intracardiac blood aspirated from the operative field by a pump flows into the cardiotomy section 900 through the conduit tube 935, and flows out of the cardiotomy section 900 after passing through the filter 910. In the cardiotomy section 900, air bubbles contained in blood float up to the blood surface. The air bubbles that have floated up to the blood surface gradually rise as they increase in number, but they are broken when they came in contact with the defoamer 920. In this way, the defoamer 920 restricts the growth of air bubbles on the blood surface so that the air bubbles do not rise above a certain level.

Patent Document 1 discloses that the lower end of the conduit tube 935 should be located at a higher level than the maximum blood surface level 950, and more preferably, at a higher level than the lower end of the defoamer 920, because the number of generated air bubbles will increase if the lower end of the conduit tube 935 becomes immersed in blood in the cardiotomy section.
Patent Document 1: JP2002-165878A (paragraph [0040] and FIG. 2)

### Disclosure of Invention

### Problem to be Solved by the Invention

The conventional cardiotomy section 900 illustrated in FIG. 20, however, has the problem of poor defoaming performance. This is because a space 960 surrounded by the generally cylindrical defoamer 920 is formed under the conduit tube 935. Air bubbles on the blood surface can grow to rise within this space 960, which consequently causes a problem that atmospheric pressure within the cardiotomy section increases, and the resistance to the inflow of blood or a liquid medicine into the cardiotomy section increases accordingly.

It is thus an object of the present invention to solve the above-described conventional problems and to provide a blood reservoir equipped with a cardiotomy section having high defoaming performance.

### Means for Solving Problem

A blood reservoir according to the present invention includes a housing that includes an intracardiac blood inflow port in an upper portion thereof and a blood outflow port at a lower end thereof, a cardiotomy section arranged in the housing, and a conduit tube that communicates with the intracardiac blood inflow port and allows blood to flow out of the intracardiac blood inflow port into the cardiotomy section. The conduit tube is inserted from above downward in the cardiotomy section. The cardiotomy section includes a filter and a defoamer arranged inside the filter. A lower end of the filter is sealed or folded in an approximately straight line or in a curve. As viewed from above, the defoamer is arranged annularly along an inner peripheral surface of the filter.

A lower end of the conduit tube is located at the same level as or at a lower level than a lower end of the defoamer, with respect to a vertical direction.

In a projection view of a lower end face of the defoamer with respect to a horizontal plane, (Ru2-2×Tu)/(Ru1-2×Tu)≤0.24 is satisfied where Ru1 is an outer dimension of the annularly arranged defoamer in a major axial direction, Ru2 is an outer dimension thereof in a minor axial direction, and Tu is a thickness of the defoamer.

### Effects of the Invention

The present invention achieves a blood reservoir equipped with a cardiotomy section having high defoaming performance.

### Brief Description of Drawings

[FIG. 1] FIG. 1 is a perspective view illustrating a general configuration of a blood reservoir according to Embodiment 1 of the present invention.
[FIG. 2] FIG. 2 is a cross-sectional side view illustrating a general configuration of the blood reservoir according to Embodiment 1 of the present invention.
[FIG. 3] FIG. 3 is a perspective view of a support member provided in the interior of the blood reservoir illustrated in FIG. 1, according to Embodiment 1 of the present invention.
[FIG. 4A] FIG. 4A is a side view of a cardiotomy section used in the blood reservoir according to Embodiment 1 of the present invention.
[FIG. 4B] FIG. 4B is a cross-sectional view taken along the line 4B-4B in FIG. 4A.
[FIG. 4C] FIG. 4C is a cross-sectional view taken along the line 4C-4C in FIG. 4A.
[FIG. 5A] FIG. 5A is a perspective view of a filter that configures the cardiotomy section according to Embodiment 1 of the present invention.
[FIG. 5B] FIG. 5B is a side view of the filter illustrated in FIG. 5A.
[FIG. 5C] FIG. 5C is a top view of the filter illustrated in FIG. 5A.
[FIG. 6] FIG. 6 is a perspective view illustrating a step for manufacturing a filter that configures the cardiotomy section according to Embodiment 1 of the present invention.
[FIG. 7] FIG. 7 is a perspective view illustrating a step for manufacturing a filter that configures the cardiotomy section according to Embodiment 1 of the present invention.
[FIG. 8A] FIG. 8A is a cross-sectional view illustrating that a conduit tube is inserted in the cardiotomy section used in the blood reservoir according to Embodiment 1 of the present invention.
[FIG. 8B] FIG. 8B is a cross-sectional view taken along the line 8B-8B in FIG. 8A.
[FIG. 9A] FIG. 9A is a side view of a cardiotomy section used in a blood reservoir according to Embodiment 2 of the present invention.
[FIG. 9B] FIG. 9B is a cross-sectional view taken along the line 9B-9B in FIG. 9A.
[FIG. 9C] FIG. 9C is a cross-sectional view taken along the line 9C-9C in FIG. 9A.
[FIG. 10A] FIG. 10A is a perspective view of a filter that configures the cardiotomy section according to Embodiment 2 of the present invention.
[FIG. 10B] FIG. 10B is a side view of the filter illustrated in FIG. 10A
[FIG. 10C] FIG. 10C is a top view of the filter illustrated in FIG. 10A.
[FIG. 11] FIG. 11 is a perspective view illustrating a step for manufacturing a filter that configures the cardiotomy section according to Embodiment 2 of the present invention.
[FIG. 12A] FIG. 12A is a perspective view illustrating a step for manufacturing a filter that configures the cardiotomy section according to Embodiment 2 of the present invention.
[FIG. 12B] FIG. 12B is a perspective view illustrating a step for manufacturing a filter that configures the cardiotomy section according to Embodiment 2 of the present invention.
[FIG. 13A] FIG. 13A is a perspective view illustrating a step for manufacturing a filter that configures the cardiotomy section according to Embodiment 2 of the present invention.
[FIG. 13B] FIG. 13B is a perspective view illustrating a step for manufacturing a filter that configures the cardiotomy section according to Embodiment 2 of the present invention.
[FIG. 14] FIG. 14 is a side view of another cardiotomy section used in the blood reservoir according to Embodiment 2 of the present invention.
[FIG. 15] FIG. 15 is a cross-sectional side view illustrating a general configuration of a blood reservoir equipped with the cardiotomy section illustrated in FIG. 14, according to Embodiment 2 of the present invention.
[FIG. 16] FIG. 16 is a perspective view illustrating slits formed at the lower end of a conduit tube that allows the inflow of intracardiac blood into a cardiotomy section in a blood reservoir according to Embodiment 3 of the present invention.
[FIG. 17] FIG. 17 is a cross-sectional view illustrating that the lower end of the conduit tube that allows the inflow of intracardiac blood into the cardiotomy section becomes immersed in blood in the blood reservoir according to Embodiment 3 of the present invention.
[FIG. 18] FIG. 18 is a diagram explaining the reason why negative pressure builds up in a liquid medicine injection port that is connected to the conduit tube that allows the inflow of intracardiac blood into the cardiotomy section in the blood reservoir according to Embodiment 3 of the present invention.
[FIG. 19] FIG. 19 is a perspective view illustrating through holes formed in the vicinity of the lower end of the conduit tube that allows the inflow of intracardiac blood into the cardiotomy section in another blood reservoir according to Embodiment 3 of the present invention.
[FIG. 20] FIG. 20 is a cross-sectional view illustrating an example of a general configuration of a conventional cardiotomy section.

### Description of the Invention

### Embodiment 1

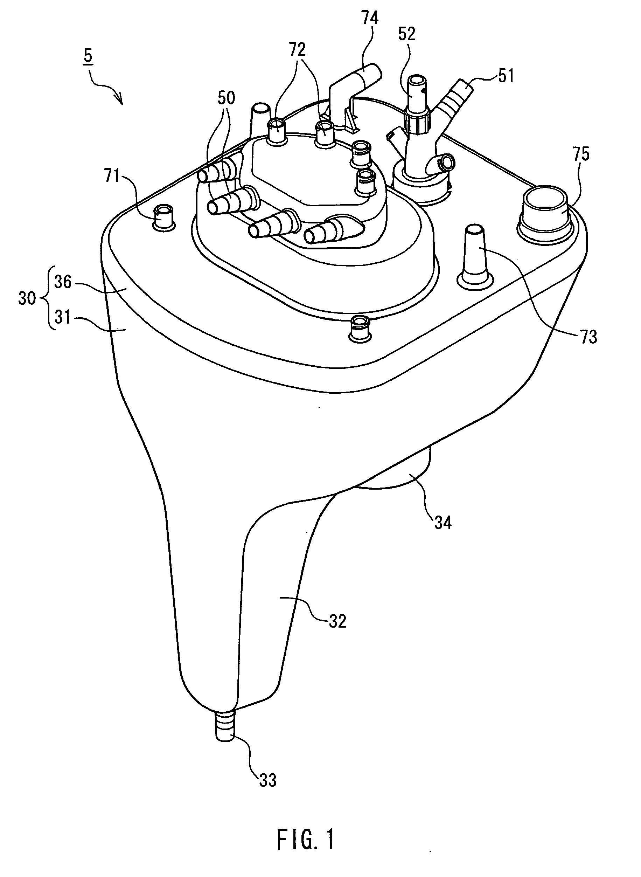

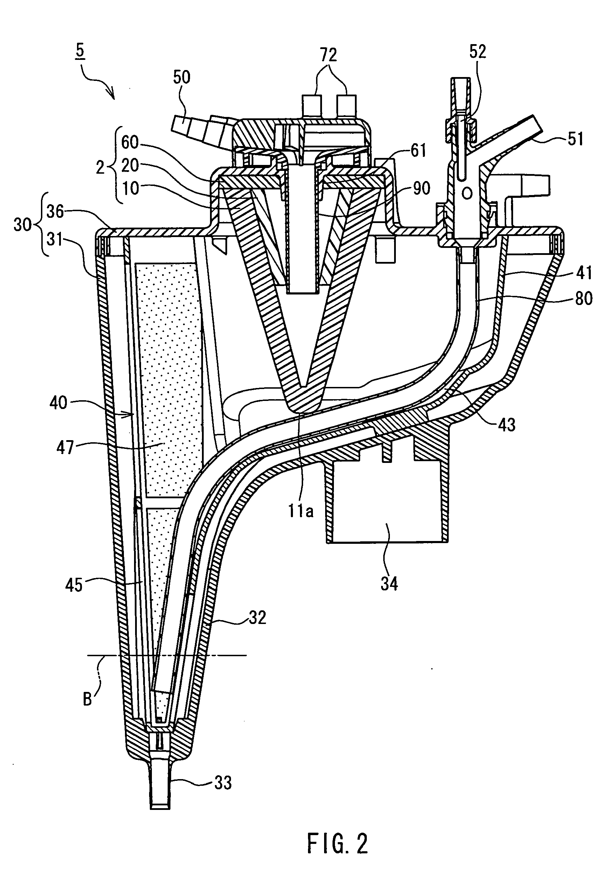

FIG. 1 is a perspective view illustrating a general configuration of a blood reservoir 5 according to Embodiment 1 of the present invention, and FIG. 2 is a cross-sectional side view thereof. The blood reservoir 5 includes a housing 30 that is composed of a housing body 31 and a lid 36 placed on the top of the housing body 31.

The housing body 31 includes a blood storage section 32 that is formed by a non-central part of the bottom protruding downward, and a blood outflow port 33 that is provided at the lower end of the blood storage section 32 and allows the outflow of blood. A fixing hole 34 is formed on the underside of the housing body 31 in order to hold the blood reservoir 5 by inserting therein an upper end of a support provided upright in an operating room.

The lid 36 has mounted thereon multiple intracardiac blood inflow ports 50 that allow the inflow of intracardiac blood, and a venous blood inflow port 51 that allows the inflow of venous blood. The lid 36 further is provided with multiple liquid medicine injection ports 71 and 72 for mixing a liquid medicine or the like in blood, a service port 73 for mixing a large volume of liquid medicine into blood in an urgent need or for allowing the inflow of blood that has passed through an alternate cardiotomy section in a case where the filter 10 of the cardiotomy section 2 cannot be used because of clogging, an evacuation port 74 for adjusting pressure in the blood reservoir 5, a pressure regulating valve 75 for preventing development of an abnormal positive or negative pressure in the blood reservoir 5, and so on. The venous blood inflow port 51 has a temperature probe 52 stuck therein for measuring the temperature of venous blood.

The venous blood inflow port 51 is connected to a blood removal line tube in an extracorporeal circulation circuit, and the intracardiac blood inflow ports 50 are connected to intracardiac blood aspiration line tubes. The blood outflow port 33 is connected to a blood sending line tube in the extracorporeal circulation circuit. The liquid medicine injection ports 71 and 72 are connected to liquid medicine injection line tubes connected to predetermined liquid medicine packs. A liquid medicine from the liquid medicine injection port 71 flows into the blood storage section 32 without passing through the cardiotomy section 2, whereas a liquid medicine from the liquid medicine injection port 72 flows into the blood storage section 32 after passing through the cardiotomy section 2. The service port 73 is connected to various line tubes. The temperature probe 52 is connected to electric wiring connected to temperature measuring equipment.

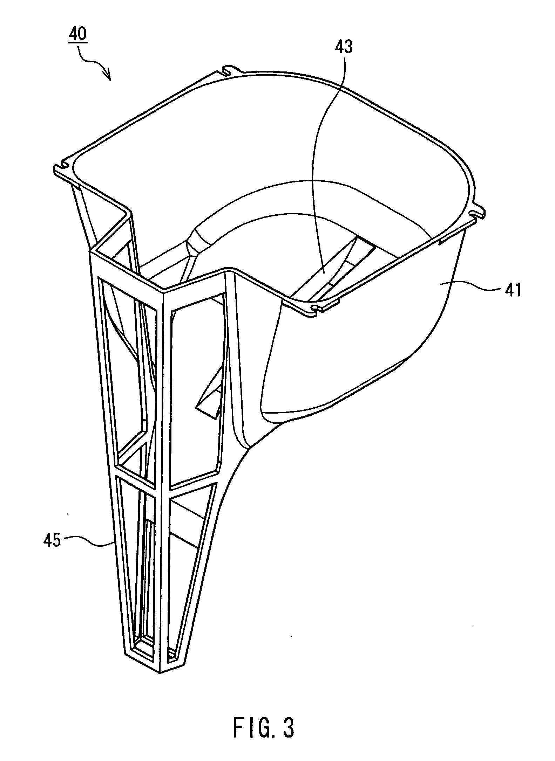

The housing 30 stores therein a support member 40 for holding a venous blood filter screen 47. FIG. 3 is a perspective view illustrating the support member 40 without holding the venous blood filter screen 47. The support member 40 includes a generally square-shaped cup section 41 and a frame section 45 composed of a lattice frame formed on one side of the cup section 41. A groove 43 is formed in the bottom of the cup section 41. The frame section 45 extends downward below the cup section 41 so that it can be inserted in the blood storage section 32 of the housing body 31. The venous blood filter screen 47 is fixed and held against the frame section 45 so as to close the openings formed on the sides of the frame section 45. The openings of the frame section 45 extend over substantially the entire vertical range of the frame section 45, with their lower ends reaching the vicinity of the blood outflow port 33.

The venous blood filter screen 47 has no particular limitations on its configuration and material as long as it has the function of a filter for removing extraneous materials or air bubbles in blood, and any known material or the like may be selected and used as appropriate. For example, a screen filter having a large number of superfine openings may be used as the venous blood filter screen 47.

The venous blood inflow port 51 and the upper end of a venous blood inlet tube 80 are connected via the lid 36. The venous blood inlet tube 80 is fitted into the groove 43 of the cup section 41 and guided inside the support member 40 from the cup section 41 to the frame section 45, with a lower end opening thereof located at a lower level than a minimum blood surface level B in the blood reservoir 5.

Below the intracardiac blood inflow port 50, the cardiotomy section 2 is arranged in the support member 40. The cardiotomy section 2 includes the filter 10 having a bag-like shape as a whole (or a shape similar to a paper coffee filter used to extract coffee), a defoamer 20 arranged inside the filter 10, and a resin plate 60 bonded to the upper ends of the filter 10 and the defoamer 20. The resin plate 60 has a through hole 61 formed in about the center thereof. A conduit tube 90 is inserted from above in the through hole 61. The conduit tube 90 communicates with the multiple intracardiac blood inflow ports 50 and the multiple liquid medicine injection ports 72 provided on the lid 36. Alower end (folded part) 11a of the filter 10 is in contact with the bottom of the cup section 41, which reduces foaming of the blood flowing out of the cardiotomy section 2.

The flow of blood in the blood reservoir 5 is described below briefly. The venous blood removed from a patient's vein passes through the venous blood inflow port 51 and the venous blood inlet tube 80 in sequence, flows out of the lower end opening of the venous blood inlet tube 80, passes through the venous blood filter screen 47, and flows out of the blood outflow port 33. Meanwhile, the intracardiac blood aspirated from a patient's operative field passes through an intracardiac blood inflow port 50, the conduit tube 90, and the cardiotomy section 2 in sequence, flows into the support member 40, passes through the venous blood filter screen 47, and flows out of the blood outflow port 33. In the course of this process, blood is stored temporarily in the blood storage section 32.

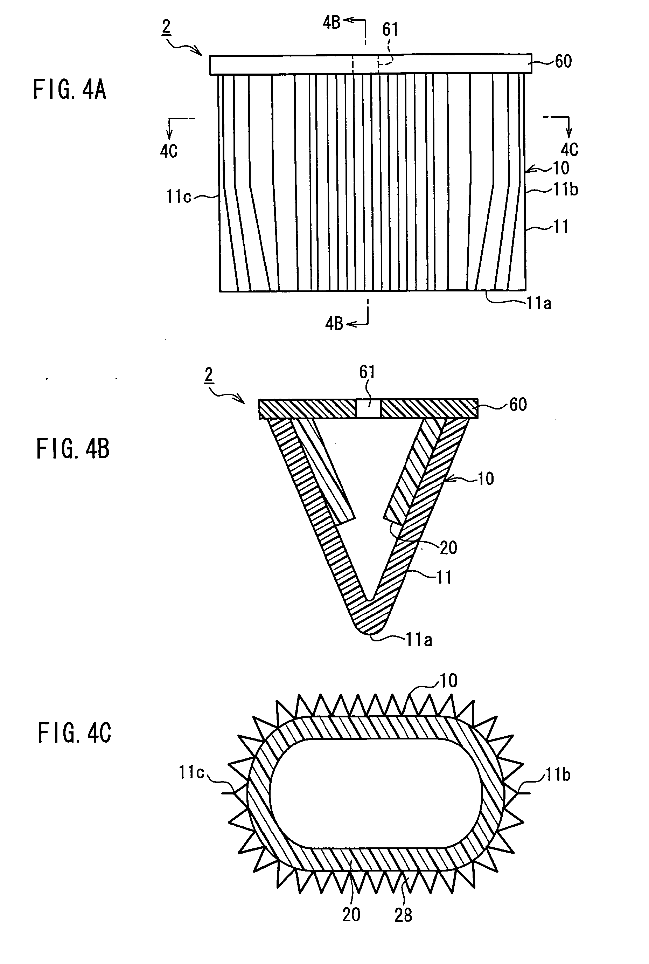

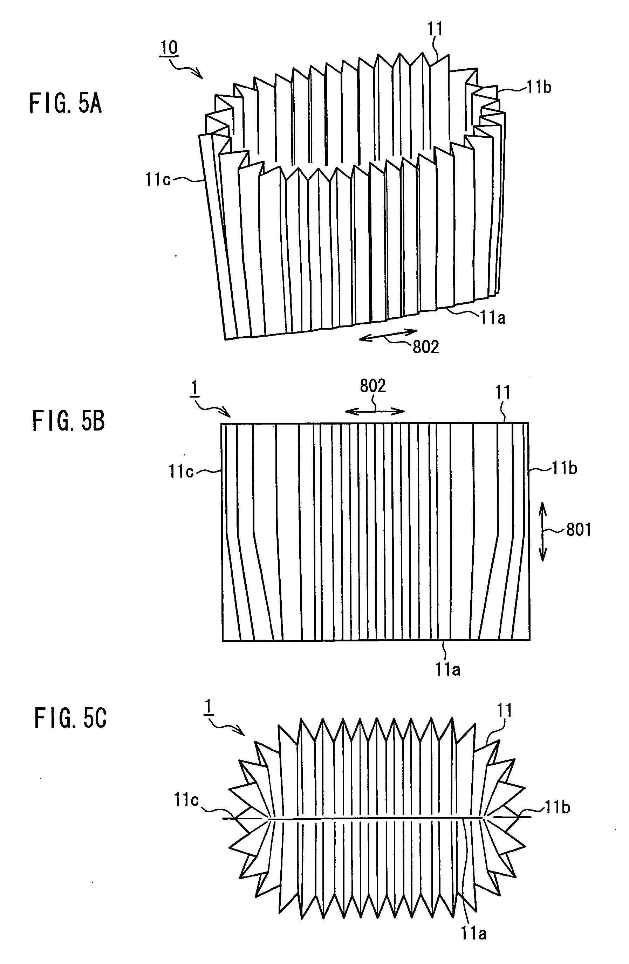

FIG. 4A is a side view of the cardiotomy section 2, FIG. 4B is a cross-sectional view taken along the line 4B-4B in FIG. 4A, and FIG. 4C is a cross-sectional view taken along the line 4C-4C in FIG. 4A. FIG. 5A is a perspective view of the filter 10 that configures the cardiotomy section 2, FIG. 5B is a side view thereof, and FIG. 5C is a top view thereof. The filter 10 is made of a filter member 11 that is pleated along a first direction 801 in FIG. 5B. The filter member 11 has a folded part 11a that is folded along a second direction 802 intersecting with the first direction 801. The filter member 11 also is sealed at a pair of sealed edges 11b and 11c thereof that intersect with the second direction 802.

A method for manufacturing such a filter 10 is described below.

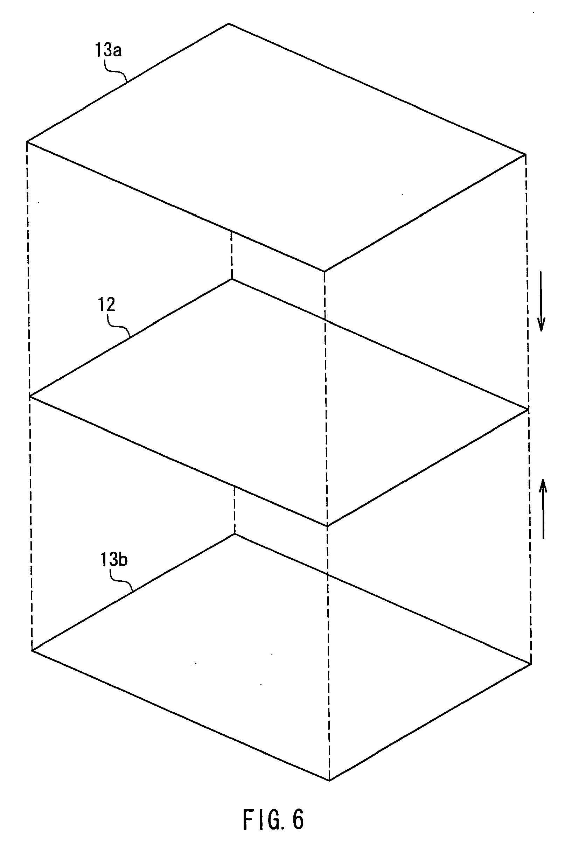

First, as illustrated in FIG. 6, support members 13a and 13b are laminated on opposite sides of a screen filter 12, which forms the rectangular filter member 11 having a three-layered laminate structure.

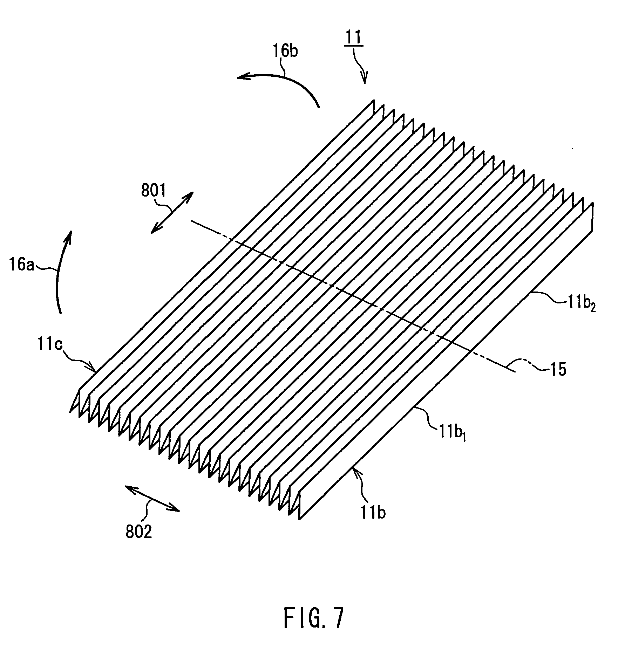

Then, as illustrated in FIG. 7, multiple pleats are formed along the first direction 801 that is parallel to one side of the rectangular filter member 11. Specifically, mountain folds and valley folds are repeated at a constant pitch along a direction parallel to the first direction 801.

The filter member 11 then is folded in the direction indicated by the arrows 16a and 16b along a fold line 15 indicated by the chain double-dashed line parallel to the second direction 802 orthogonal to the first direction 801, the fold line 15 passing through an intermediate position of the filter member 11 in the first direction 801. At this time, the filter member 11 can be folded with ease and good appearance, for example if a straight-line edge of a jig made of a hard material such as resin or metal is pressed against the filter member 11 along the fold line 15, with all the mountains (ridges) of the pleats that are in contact with the jig being displaced toward either side of the second direction 802.

Then, both edges of the filter member 11 with respect to the second direction 802 are sealed and bonded together. Specifically, referring to an edge 11b on one side with respect to the second direction 802, an edge portion 11b₁ on one side with respect to the fold line 15 and a short edge portion 11b₂ on the other side are sealed overlapping each other. An edge 11c on the other side with respect to the second direction 802 is also sealed in a similar manner. The method of sealing is not particularly limited and may be selected as appropriate taking into consideration the material for the filter member 11 or the like, and for example, a heat seal method may be used. At this time, a material such as vinyl chloride that improves sealing properties may be inserted between the two sealed members (e.g., between the edge portions 11b₁ and 11b₂).

In this manner, the filter 10 having a bag-like shape as a whole (a shape similar to a paper coffee filter used to extract coffee), as illustrated in FIGS. 5A to 5C, is obtained.

The filter member 11 of the filter 10 has a three-layered laminate structure composed of the screen filter 12 and the pair of support members 13a and 13b that sandwich the screen filter 12. Because the screen filter 12 is held by being sandwiched between the pair of support members 13a and 13b having relatively high mechanical strength, the screen filter 12 can be maintained in a desired shape. In addition, since multiple pleats are formed in the filter member 11, the surface area of the filter member 11 increases, which improves the filtration efficiency and prolongs the life of the filter.

The screen filter 12 has the function of catching and removing extraneous materials in blood when the blood passes through. It further may have the function of catching air bubbles. There are no particular limitations on the screen filter 12 having such a function, and any known screen filter used in conventional cardiotomy sections may be selected and used arbitrarily. For example, a mesh filter composed of a resin material such as polyester, nylon, or polypropylene may be employed. The hole diameter of such a filter is not particularly limited, but is preferably from 20 µm to 50 µm inclusive.

The support members 13a and 13b are used to maintain the shape of the screen filter 12. They thus need to have higher mechanical strength than the screen filter 12. There are no particular limitations on the support members 13a and 13b, and any known support member used in a conventional cardiotomy section may be selected and used arbitrarily. For example, a mesh member composed of a material having good heat-sealing properties, such as polypropylene, may be employed. The hole diameter of the support members 13a and 13b is preferably larger than the hole diameter of the screen filter 12.

The screen filter 12 and/or the support members 13a and 13b may be coated with a defoaming agent (e.g., silicone) so that they have the function of defoaming.

In FIGS. 4A to 4C, the resin plate 60 is bonded around the entire perimeter to the edge of the filter 10 on the opposite (upper) side of the filter 10 from the folded part 11a. The material for the resin plate 60 is not particularly limited; it may be an adhesive such as polyurethane, for example. In the example illustrated in FIGS. 4A and 4B, the resin plate 60 has an oval shape in plane (i.e., tracks in an athletic field). However, the shape is not limited thereto and may be selected from any arbitrary shape such as an ellipse, an circle, or a rectangle. The provision of the resin plate 60 improves the shape-keeping property of the filter 10. The resin plate 60 has formed therein the through hole 61 that allows the inflow of blood.

The defoamer 20 is, as illustrated in FIG. 4C, provided annularly along the inner peripheral face of the filter 10 inside the filter 10. The pleating of the filter 10 creates gaps 28 between the filter 10 and the defoamer 20. As illustrated in FIG. 4B, the defoamer 20 is provided in only an upper region of the filter 10 with respect to the vertical direction, and the defoamer 20 is held against the resin plate 60 by the upper edge thereof bonded to the resin plate 60. There are no particular limitations on the defoamer 20 as long as it has the function of breaking air bubbles that have come in contact, and any known defoamer used in a conventional cardiotomy section may be selected and used arbitrarily. For example, a material whose polyurethane surface as a substrate is coated with silicone oil as a defoaming agent may be employed. The form of the anti-forming material 20 may be an open-cell foam, fabric, knitted fabric, or non-woven fabric, for example. The defoamer 20 may be a single layer or may have a two or more layered laminate configuration.

The cardiotomy section 2 according to Embodiment 1 of the present invention does not include the lower resin plate 932 of the conventional cardiotomy section 900 illustrated in FIG. 20. Accordingly, it has excellent initial permeability and a secondary effect of reducing priming volume and remaining blood volume. Here, the initial permeability is determined by the volume of liquid filler that is required for the liquid filler to start to flow out of the filter 10 when the liquid filler first flows into the cardiotomy section 2 through the conduit tube 90. Excellent initial permeability reduces the blood volume, i.e., a circuit filling volume that is required to fill in the extracorporeal circulation circuit. Such reduced circuit filling volume reduces the volume of blood that is transferred from a patient's body to outside the body, thus reducing the burden on the patient. The priming volume is a total volume of a static filling volume that exists before circulation and a redundant fluid volume necessary for circulation, and it also includes the volume of fluid left in the filter 10 or the like. A low priming volume not only reduces the circuit filling volume but also improves responsiveness to fluctuations in the blood volume in the blood reservoir, thus reducing an operator workload at the time of controlling and adjusting the blood surface level. Remaining blood refers to blood that is left in the cardiotomy section 2 after the termination of extracorporeal circulation of blood. With a low remaining blood volume, the volume of blood that is returned to a patient increases, and the burden on the patient is reduced accordingly.

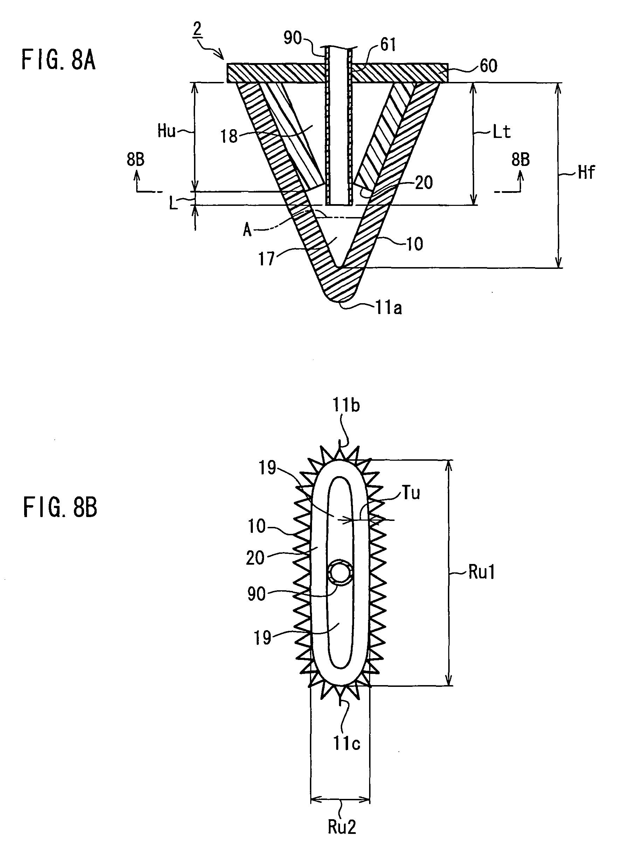

FIG. 8A is a cross-sectional view taken along the center line of the conduit tube 90, showing that the conduit tube 90 is inserted in the through hole 61 of the resin plate 60 of the cardiotomy section 2, and FIG. 8B is a cross-sectional view taken along the line 8B-8B in FIG. 8A. The chain double-dashed line A indicates a blood surface level in the cardiotomy section 2, and the blood surface level ordinarily is located at a lower level than the lower end of the conduit tube 90.

As illustrated in FIG. 8A, the lower end of the conduit tube 90 is located at the same level as or at a lower level than the lower end of the defoamer 20. In other words, Lt/Hu≥ 1 is satisfied (hereinafter referred to as a "condition A"), where Lt is the length of protrusion of the conduit tube 90 into the cardiotomy section 2 (i.e., the vertical distance from the underside of the resin plate 60 to the lower end of the conduit tube 90) and Hu is the vertical dimension of the defoamer 20 (i.e., the vertical distance from the underside of the resin plate 60 to the lower end of the defoamer 20).

Also, as illustrated in FIG. 8B, in a projection view of the lower end face of the defoamer 20 with respect to a horizontal plane, (Ru2-2×Tu)/(Rul-2×Tu)≤0.24 is satisfied (hereinafter referred to as a "condition B"), where Ru1 is the outer dimension of the defoamer 20 in a major axial direction, Ru2 is the outer dimension thereof in a minor axial direction, and Tu is the thickness of the defoamer 20. Preferably, (Ru2-2×Tu)/(Ru1-2×Tu)≤0.18 is satisfied. In the above projection view, the major axial direction of the defoamer 20 refers to a direction in which the defoamer 20 has the maximum outer dimension and which is parallel to the folded part 11a, and the minor axial direction refers to a direction orthogonal to the major axial direction. When the defoamer 20 is compressed and deformed in contact with the conduit tube 90 or the facing defoamer 20, the thickness Tu indicates a thickness without being compressed.

The above-described condition A implies that the lower end of the defoamer 20 surrounds the conduit tube 90. In the above-described condition B, the ratio (Ru2-2x Tu)/(Ru1-2×Tu) represents the aspect ratio of a region that is surrounded by the ring-shaped defoamer 20 as viewed upward from a space (lower space) 17 located at a lower level than the lower end of the defoamer 20 in the filter 10 (see FIG. 8B). The above-described condition B thus implies that, in the above projection view, the ring-shaped defoamer 20 is deformed into an oblate figure and that, in the filter 10, a channel 19 that connects the lower space 17 at a lower level than the defoamer 20 and a space (upper space) 18 at an upper level has a small area or does not exist substantially. Air bubbles contained in the blood that flows out of the lower-end opening of the conduit tube 90 into the lower space 17 float up to the blood surface and gradually grow so as to rise as they increase in number. However, because almost all the upper area above the lower space 17 is covered with the defoamer 20 and the conduit tube 90, many air bubbles are broken upon coming in contact with the lower end of the defoamer 20. This accordingly improves the defoaming performance of the cardiotomy section 2.

If L is the vertical distance between the lower end of the conduit tube 90 and the lower end of the defoamer 20, it is preferable that L/Hu≤0.1 be satisfied. If the ratio L/Hu is higher than this upper limit, i.e., if the length L of downward protrusion of the conduit tube 90 from the lower end of the defoamer 20 is too long, the possibility that the lower end of the conduit tube 90 is immersed in the blood stored in the cardiotomy section 2 is increased, which increases the resistance to the inflow of blood into the cardiotomy section 2 through the conduit tube 90.

If Hf is the inner vertical dimension of the filter 10 (i.e., the vertical distance from the underside of the resin plate 60 to the lower end of the inner wall of the filter 10), it is preferable that 0.2≤Hu/Hf≤0.95, or more preferably 0.3≤Hu/Hf≤0.9, be satisfied. If the ratio Hu/Hf is higher than the upper limit expressed by this inequality, the length Lt of the conduit tube 90 needs to be increased in order to satisfy the above condition A. This consequently increases the possibility that the lower end of the conduit tube 90 is immersed in the blood stored in the cardiotomy section 2, thereby increasing the resistance to the inflow of blood into the cardiotomy section 2. On the contrary, if the ratio Hu/Hf is lower than the lower limit expressed by this inequality, the upper space 18 becomes small and the area of the defoamer 20 that surrounds the upper space 18 also becomes small accordingly. This deteriorates the defoaming property in the upper space 18, i.e., the property of breaking air bubbles that have grown up into the upper space 18 through the channel 19.

The material for the conduit tube 90 is not particularly limited, and it may be the same material as used for the conventional conduit tube 935, such as polycarbonate. Also, the dimensions of the conduit tube 90 are not particularly limited; however, the outer diameter thereof is preferably from 8 mm to 16 mm inclusive, the inner diameter thereof is preferably from 6 mm to 12 mm inclusive, and the thickness thereof is preferably from 1.0 mm to 2.0 mm inclusive.

### Embodiment 2

Embodiment 2 according to the present invention differs from Embodiment 1 with respect to the filter that configures the cardiotomy section. Now, what is different from Embodiment 1 will be described.

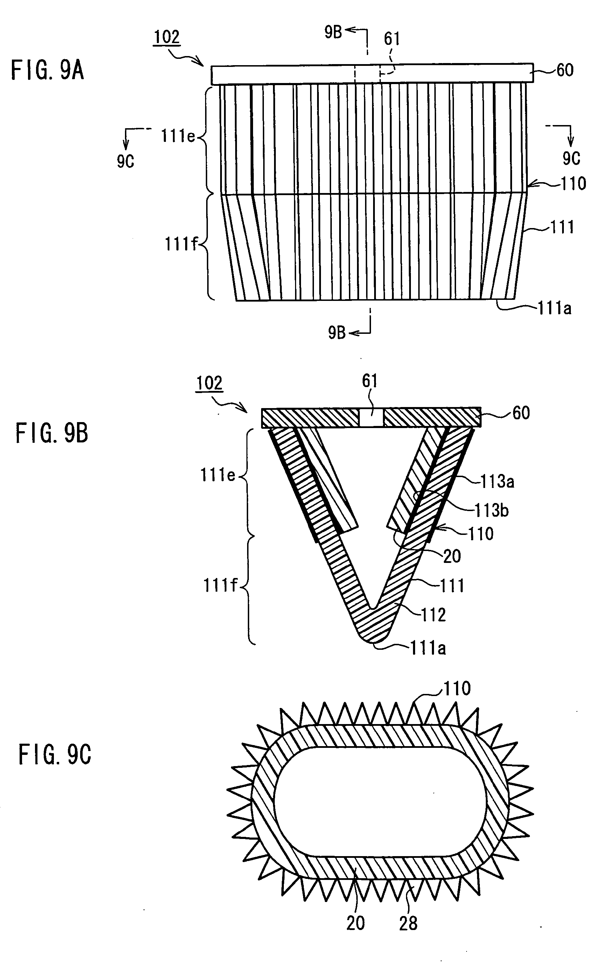

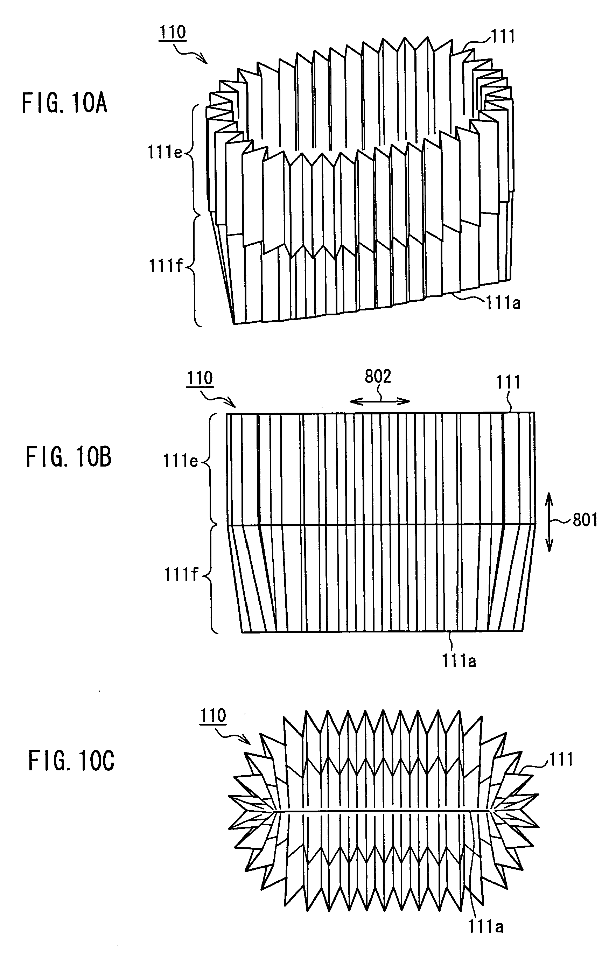

FIG. 9A is a side view of a cardiotomy section 102 according to Embodiment 2 of the present invention, FIG. 9B is a cross-sectional view taken along the line 9B-9B in FIG. 9A, and FIG. 9C is a cross-sectional view taken along the line 9C-9C in FIG. 9A. FIG. 10A is a perspective view of a filter 110 that configures the cardiotomy section 102, FIG. 10B is a side view thereof, and FIG. 10C is a top view thereof.

Like the cardiotomy section 2 according to Embodiment 1, the cardiotomy section 102 includes the filter 110 having a bag-like shape as a whole (or a shape similar to a paper coffee filter used to extract coffee), a defoamer 20 arranged inside the filter 110, and a resin plate 60 bonded to the upper ends of the filter 110 and the defoamer 20. The resin plate 60 has a through hole 61 formed at about the center thereof Like the cardiotomy section 2 according to Embodiment 1, the cardiotomy section 102 is mounted on a blood reservoir 5, with a conduit tube 90 inserted from above in the through hole 61 (see FIG. 2).

The filter 110 is made of a filter member 111 that is pleated along a first direction 801 illustrated in FIG. 10B. The filter member 111 is bonded into a tubular shape so that a second direction 802 intersecting with the first direction 801 forms a circumferential direction, and sealed along a sealing portion 111a at one edge thereof in the first direction 801. The filter member 111 has a three-layered laminate structure in a region on the side opposite from the sealing portion 111a with respect to the first direction 801, the three-layered laminated structure consisting of a screen filter and a pair of support members that sandwich the screen filter, and it also has a single layer structure composed of only a screen filter in a region on the sealing portion 111a side with respect to the first direction 801. Hereinafter, a region of the filter member 111 that has a three-layered laminate structure equipped with the support members is referred to as a "support portion 111e", and the other region thereof that has a single layer structure with no support member is referred to as a "non-support portion 111f".

A method for manufacturing the filter 110 is described below.

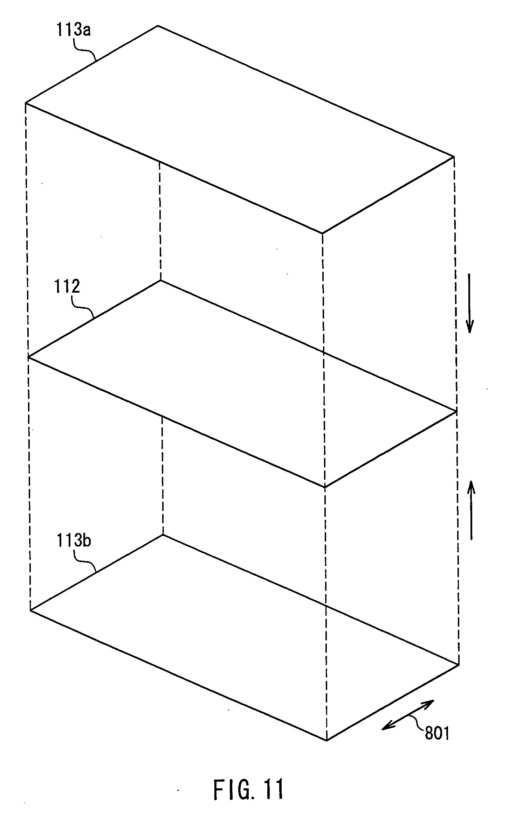

First, as illustrated in FIG. 11, support members 113a and 113b are laminated on opposite sides of a screen filter 112 so as to form a rectangular filter member material 111' halving a three-layered laminate structure.

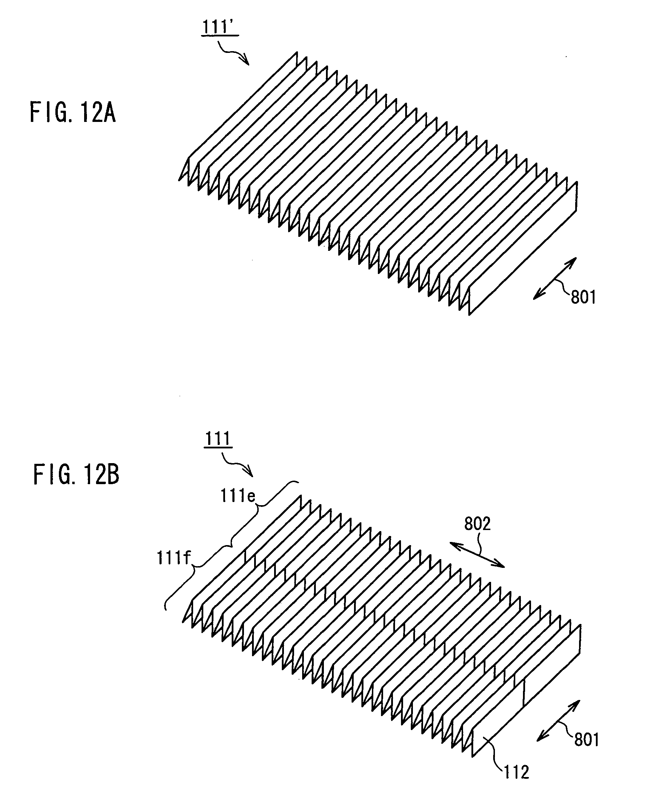

Then, as illustrated in FIG. 12A, multiple pleats are formed along the first direction 801 that is parallel to the short side of the rectangular filter member material 111'. Specifically, mountain folds and valley folds are repeated at a constant pitch along a direction parallel to the first direction 801.

Then, as illustrated in FIG. 12B, the support members 113a and 113b are cut in parallel with the second direction 802 that is parallel to the long side of the filter member material 111' so as to remove the support members 113a and 113b on one side with respect to the cut position. This creates the filter member 111 that includes the non-support portion 111f having a single layer structure composed of only the screen filter 112 on one side with respect to the first direction 801, and the support portion 111e having a three-layered laminate structure composed of the screen filter 112 and the pair of support members 113a and 113b that sandwich the screen filter 112 on the other side.

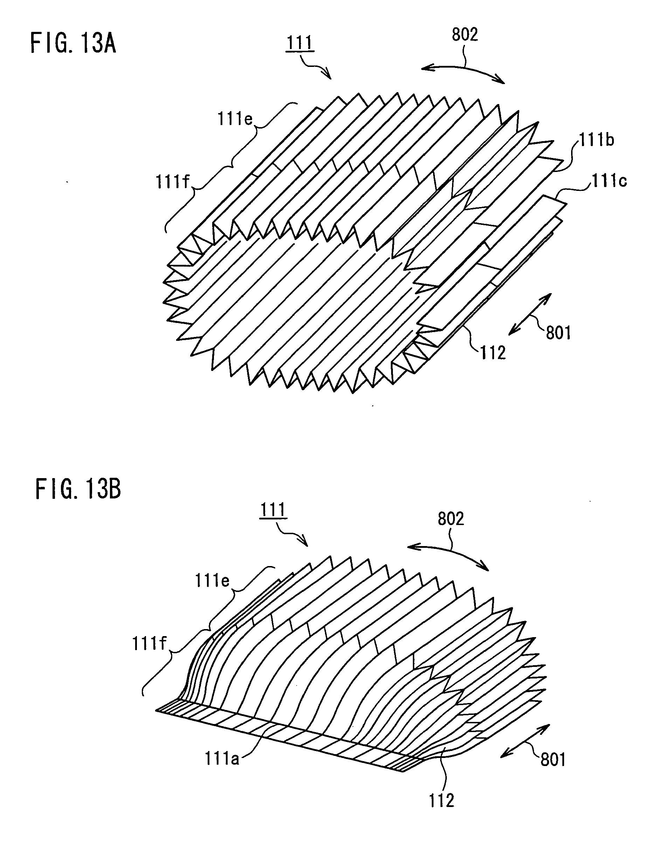

Then, one short side 111b and the other short side 111c of the first member 111 are sealed and bonded into a tubular shape as illustrated in FIG. 13A. At this time, the second direction (long-side direction) 802 illustrated in FIG. 12B is a circumferential direction of a resultant tubular object. The sealing is performed over the entire width of the filter member 111 in the first direction 801. A method for sealing the short sides 111b and 111c is not particularly limited and may be selected as appropriate taking into consideration the material for the filter member 111 or the like; for example, a heat seal method may be used. At this time, a material such as vinyl chloride that improves sealing properties may be inserted between the short sides 111b and 111c to be sealed.

Then, the edge on the non-support portion 111f side or in the vicinity thereof is sealed in an approximately straight line as illustrated in FIG. 13B. In FIG. 13B, 111a denotes a sealing portion formed in this way. As necessary, a redundant portion of the sealing portion 111a on the side opposite from the support portion 111e may be cut off. A method of sealing is not particularly limited and may be selected as appropriate taking into consideration the material for the screen filter 112 or the like; for example, a heat seal method may be used. At this time, a material such as vinyl chloride that improves sealing properties may be inserted between the members to be sealed.

As a last step, the filter member 111 is turned inside out so that the support member located inside with respect to the screen filter 112 in the state of FIG. 13B is located outside. In this way, the filter 110 having a bag-like shape as a whole (a shape similar to a paper coffee filter used to extract coffee) is created, with the sealing portion 111a located inside as illustrated in FIGS. 10A to FIG. 10C.

In the above-described manufacturing method, multiple pleats are formed in the filter member material 111' and thereafter parts of the support members 113a and 113b are removed so as to produce the filter member 111 having the support portion 111e and the non-support portion 111f; however, the present invention is not limited thereto. Alternatively, the method may be such that parts of the support members 113a and 113b are removed so as to produce the filter member 111 having the support portion 111e and the non-support portion 111f, and thereafter multiple pleats are formed in the filter member 111. As another alternative, in the step illustrated in FIG. 11, support members 113a and 113b having narrower widths than the screen filter 112 may be laminated on the screen filter 112 in order to directly produce the filter member 111 having the support portion 111e and the non-support portion 111f, which eliminates a subsequent step of removing parts of the support members 113a and 113b.

Still further, the filter member 111 may not have to be turned inside out after the formation of the sealing portion 111a.

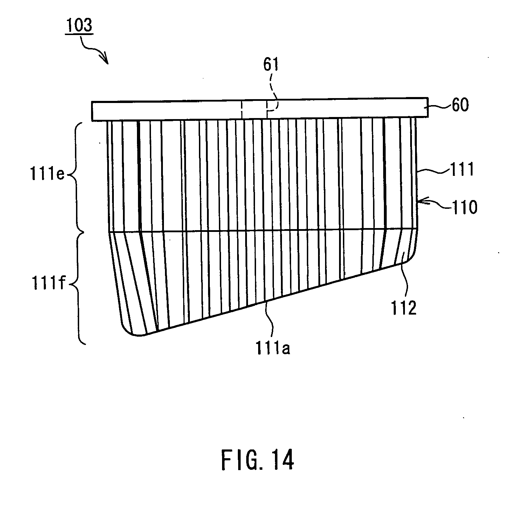

FIG. 14 is a side view of another cardiotomy section 103 according to Embodiment 2 of the present invention. The cardiotomy section 103 illustrated in FIG. 14 differs from the cardiotomy section 102 illustrated in FIGS. 9A to 9C in that the approximately straight-line sealing portion 111a is tilted with respect to the resin plate 60. The tilt angle of the sealing portion 111a may be set arbitrarily. In FIG. 14, those members that have the same functions as those illustrated in FIGS. 9A to 9C are denoted by the same reference numerals.

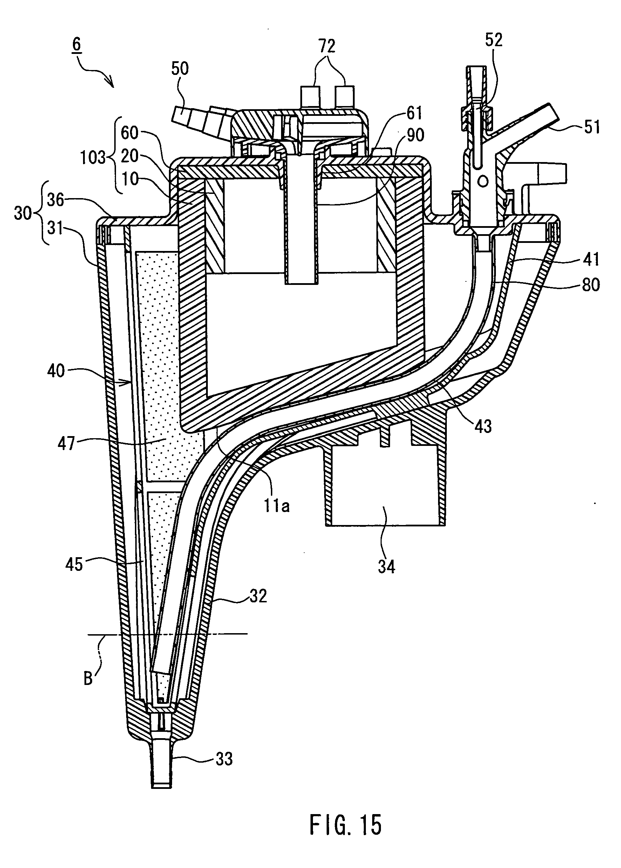

FIG. 15 is a cross-sectional side view illustrating a general configuration of a blood reservoir 6 equipped with the cardiotomy section 103 illustrated in FIG. 14, according to Embodiment 2 of the present invention.

In the cardiotomy section 2 illustrated in FIGS. 4A to 4C and the cardiotomy section 102 illustrated in FIGS. 9A to 9C, their lower ends 11a and 111a are almost parallel to the resin plate 60. Thus, as illustrated in FIG. 2, the cardiotomy sections 2 and 102 are arranged in the blood reservoir 5 so that their lower ends 11a and 111a extend in a direction perpendicular to the plane of the drawing in FIG. 2. Hence, almost all the area of the lower ends 11a and 111a are in contact with the bottom of the cup section 41.

On the other hand, in the cardiotomy section 103 illustrated in FIG. 14, the approximately straight-line sealing portion 111a at the lower end thereof is formed at an angle relative to the resin plate 60. Thus, as illustrated in FIG. 15, the cardiotomy section 103 is arranged in the blood reservoir 6 so that the sealing portion 111a extends in a direction parallel to the plane of the drawing in FIG. 15 and along the tilt of the bottom of the cup section 41 (or the venous blood inlet tube 80 fitted in the groove 43). Hence, almost all the area of the sealing portion 111a is in contact with the tilted bottom of the cup section 41 or the tilted venous blood inlet tube 80. The tilt angle of the sealing portion 111a preferably may be set according to the tilt angle of the bottom of the cup section 41 or the venous blood inlet tube 80 so that the area of contact between the sealing portion 111a and the bottom of the cup section 41 or the venous blood inlet tube 80 is as large as possible.

In other respects, Embodiment 2 of the present invention is identical to Embodiment 1.

### Embodiment 3

Embodiment 3 according to the present invention differs from Embodiment 1 with respect to the lower end of the conduit tube 90 and the configuration in the vicinity thereof. Now, only what is different from Embodiment 1 will be described.

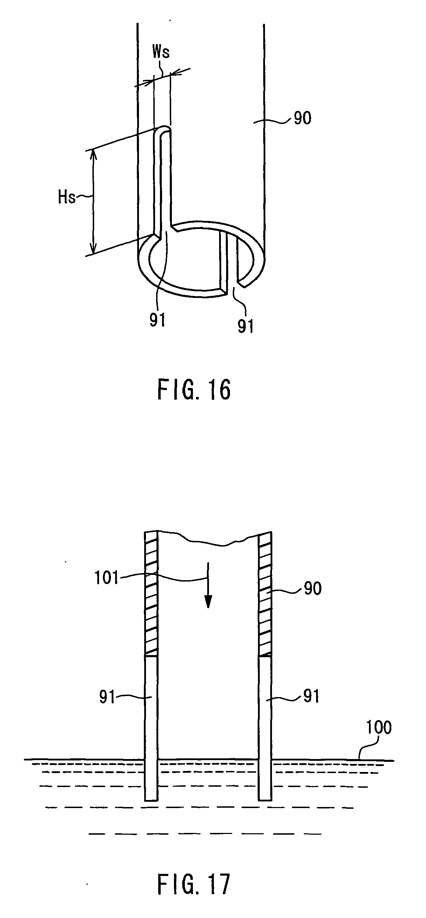

FIG. 16 is a perspective view of the lower end of the conduit tube 90 as viewed from below. In the present embodiment, as illustrated in FIG. 16, a pair of slits 91 that extend upward from the lower end of the conduit tube 90 are formed in the side of the conduit tube 90. The pair of slits 91 are formed into the same shape and in the same dimensions at diametrically opposed positions with respect to the center axis of the cylindrical conduit tube 90.

Now, the effect achieved by the pair of slits 91 will be described.

As illustrated in FIG. 8A, the blood surface level A in the cardiotomy section 2 generally is located at a lower level than the lower end of the conduit tube 90. However, in some cases, the blood surface level may rise for some sort of reason. In such a case, as described in Embodiment 1, the lower end of the conduit tube 90 may become immersed in blood because the lower end of the conduit tube 90 is located at the same level as or at a lower level than the lower end of the defoamer 20. At this time, if no slit 91 is formed in the conduit tube 94, there is a possibility that the resistance to the inflow of blood into the cardiotomy section 2 through the conduit tube 90 may increase. On the other hand, in the present embodiment, if parts of the pair of slits 91 are exposed over the blood surface even though the lower end of the conduit tube 90 becomes immersed in blood 100 as illustrated in FIG. 17, it is possible for blood (intracardiac blood) 101 flowing through the conduit tube 90 to flow out of the conduit tube 90 through the pair of slits 91, and it is also possible to prevent an increase in atmospheric pressure in the conduit tube 90. This accordingly suppresses an increase in the resistance to the inflow of blood into the cardiotomy section 2 through the conduit tube 90. By forming the slits 91 in the conduit tube 90 in this way, it becomes possible to cope with, without problems, the case where the blood surface level rises above the lower end of the conduit tube 90.

Moreover, the following effect also can be achieved if the conduit tube 90 communicates with not only the intracardiac blood inflow port 50 but also the liquid medicine injection port 72 as in the blood reservoir 5 described in Embodiment 1.

In the case where the lower end of the conduit tube 90 becomes immersed in the blood 100 as illustrated in FIG. 17, it is possible to suppress an increase in the resistance to the inflow of a liquid medicine from the liquid medicine injection port 72 into the cardiotomy section 2 through the conduit tube 90, for the same reason that an increase in the resistance to the inflow of blood into the cardiotomy section 2 through the conduit tube 90 can be suppressed.

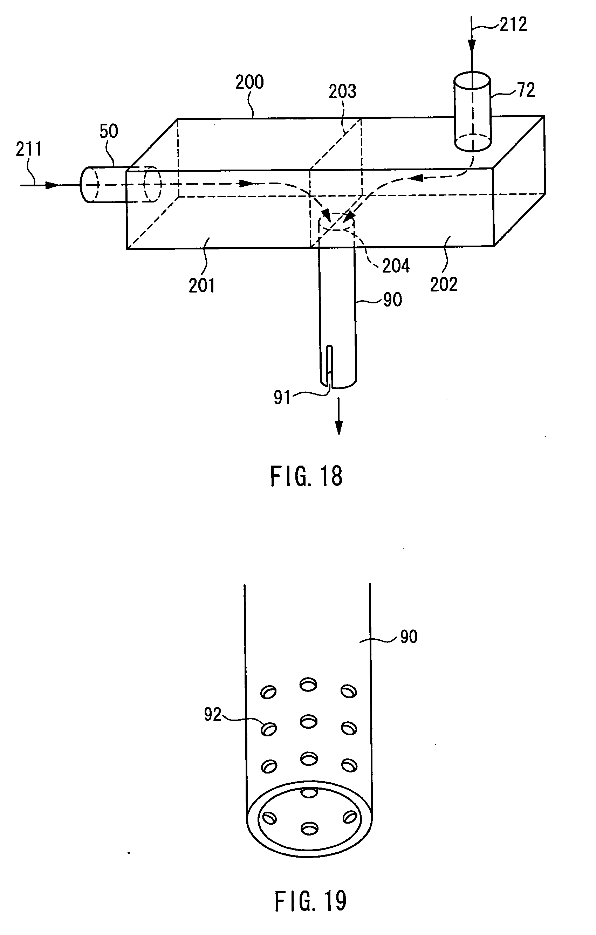

In addition, it is also possible to suppress a negative pressure from building up in the liquid medicine injection port 72 connected to the conduit tube 90 when blood flows from the intracardiac blood inflow port 50 into the cardiotomy section 2 through the conduit tube 90. This will be described with reference to FIG. 18. FIG. 18 is a perspective view illustrating a general configuration of flow channels that extend to the conduit tube 90 from the intracardiac blood inflow port 50 and the liquid medicine injection port 72. While, in order to simplify the description, FIG. 18 illustrates the case where the conduit tube 90 communicates with only a single intracardiac blood inflow port 50 and a single liquid medicine injection port 72, the same applies to the case where the conduit tube 90 communicates with multiple intracardiac blood inflow ports 50 and/or multiple liquid medicine injection ports 72. A space in a mixing vessel 200 provided on the lid 36 is divided by a partition wall 203 into a blood flow channel 201 for the flow of blood (intracardiac blood) and a liquid medicine flow channel 202 for the flow of a liquid medicine. The intracardiac blood inflow port 50 communicates with the blood flow channel 201, whereas the liquid medicine inflow port 72 communicates with the liquid medicine flow channel 202. The cylindrical conduit tube 90 is connected to an opening 204 formed in the underside of the mixing vessel 200. The partition wall 203 roughly divides the opening 204 into two sections. Blood 211 flows into the cardiotomy section 2 after passing through the intracardiac blood inflow port 50, the blood flow channel 201 and the opening 204 in the mixing vessel 200, and the conduit tube 90 in sequence. A liquid medicine 212 flows into the cardiotomy section 2 after passing through the liquid medicine injection port 72, the liquid medicine flow channel 202 and the opening 204 in the mixing vessel 200, and the conduit tube 90 in sequence. In general, the flow rate of the blood 211 is higher than the flow rate of the liquid medicine 212, so that the mixing vessel 200 forms what is called an aspirator depending on the flow of the blood 211 in the vicinity of the opening 204, and negative pressure builds up in the liquid medicine flow channel 202 accordingly. If negative pressure builds up in the liquid medicine flow channel 202, problems arise, such as the difficulty in controlling the flow rate of the liquid medicine 212. However, the presence of the slits 91 formed in the side wall of the conduit tube 90 prevents the generation of a negative pressure in the liquid medicine flow channel 202 due to the flow of the blood 211.

The length Hs of the slits 91 formed in the conduit tube 90 (the distance from the lower end of the conduit tube 90 to the upper end of the slits 91; see FIG. 16) is not particularly limited, but it is preferably from 5 mm to 30 mm inclusive, and more preferably from 10 mm to 20 mm inclusive. If the length Hs of the slits 91 is longer than the abovementioned range, the defoaming properties may deteriorate because generated air bubbles may leak out of the conduit tube 90 from the slits 91 so that they cannot effectively be in contact with the defoamer 20. On the contrary, if the length Hs of the slits 91 is shorter than the abovementioned range, it is difficult to achieve the above-described effect with the slits 91.

Also, the width W_{S} of the slits 91 formed in the conduit tube 90 (the circumferential dimension of the conduit tube 90; see FIG. 16) is not particularly limited, but it is preferably from 1 mm to 5 mm inclusive, and more preferably from 2 mm to 3 mm inclusive. If the width W_{S} of the slits 91 is wider than the abovementioned range, the defoaming properties may deteriorate for the same reason as in the abovementioned case where the length Hs of the slits 91 is too long. On the contrary, if the width W_{S} of the slits 91 is narrower than the abovementioned range, it is difficult to achieve the above-described effect with the slits 91.

In the foregoing description, while the number of slits 91 formed in the conduit tube 90 is two, the present invention is not limited thereto and the number of slits may be one or more than two. In the case where multiple slits 91 are formed, the slits 91 may preferably be arranged at regular angular intervals with respect to the center axis of the conduit tube 90. For the provision of multiple slits 91, all the slits 91 do not necessarily have the same length Hs and the same width W_{S} and may have different lengths and widths.

Instead of forming the slits 91 in the side of the conduit tube 90, through holes 92 may be formed at positions on the side of the conduit tube 90 and in the vicinity of the lower end thereof as illustrated in FIG. 19. The through holes 92 function like the slits 91, thereby achieving the same effect as achieved with the slits 91. Such through holes 92 may preferably be formed at multiple locations and arranged at diametrically opposed positions with respect to the center axis of the conduit tube 90. The positions of the through holes 92 are not particularly limited as long as they are formed in a region in the vicinity of the lower end of the conduit tube 90, but they may preferably be formed in a region on the lower end side from a location 30 mm apart from the lower end of the conduit tube 90, and more preferably a location 20 mm apart therefrom. The opening shape, opening area, number, and locations of the through holes 92 may be set as appropriate taking into consideration the volume of blood flowing through the conduit tube 90, a conceivable blood surface level, or the like. Alternatively, both the slits 91 formed as illustrated in FIG. 16 and the through holes 92 illustrated in FIG. 19 may be formed in the conduit tube 90.

In the foregoing description, while the slits 91 or the through holes 92 are provided in the conduit tube 90 in the blood reservoir according to Embodiment 1, the slits 91 or the through hole 92 may be provided similarly in the conduit tube 90 in the blood reservoir according to Embodiment 2. In that case as well, the same effect as described above can be achieved.

The above-described Embodiments 1 to 3 are merely examples and the present invention is not limited thereto.

While the above-described Embodiments 1 to 3 employ the filter 10 or 110 in which the filter member 11 or 111 including the screen filter 12 or 112 is pleated and formed into a bag-like shape, a filter of the cardiotomy section is not limited thereto. For example, a filter in which a non-woven fabric is formed into a bag-like shape without pleating may be used.

While all the lower ends 11a and 111a of the above-described cardiotomy sections 2, 102, and 103 are formed in a straight line, the present invention is not limited thereto, and they may be formed in a curve, for example. The lower ends 11a and 111a may preferably be formed in conformity with the surface shape of the member in the blood reservoir (e.g., the bottom of the cup section 41 or the venous blood inlet tube 80) so that, when the cardiotomy section is mounted in the blood reservoir, the area of contact between the lower ends 11a, 111a and the member is as large as possible. Such an increase in the area of contact between the lower ends 11a, 111a and the member in the blood reservoir allows the blood from the cardiotomy section to flow over the lower end 11a or 111a and the member in contact therewith to reach the blood storage section 32, thus preventing the foaming of blood flowing out of the cardiotomy section.

In the above-described cardiotomy sections 2, 102, and 103, although the defoamer 20 is held by being bonded to the resin plate 60, the method for holding the defoamer 20 is not limited thereto. For example, the defoamer 20 may be held with a jig that is provided at the lower end of the defoamer 20 so as to prevent the defoamer 20 from moving down with respect to the filter 10 or 110.

The blood reservoir according to the present invention is not limited to an integral type of an cardiotomy reservoir and a venous blood reservoir, such as the above-described blood reservoir 5, that allow the inflow of both venous blood and intracardiac blood, it may be any other known blood reservoir. For example, it may be an cardiotomy reservoir that does not allow the inflow of venous blood.

### Examples

### Experiment 1

A cardiotomy section that has the same structure as the cardiotomy section 2 described in Embodiment 1 was created as follows.

A mesh filter made of polyethylene terephthalate (PET) and having a hole diameter of 40 µm was used as the screen filter 12. A mesh made of polypropylene and having a hole diameter of 1 mm was used as the support members 13a and 13b. Adjacent mountain and valley portions of multiple pleats formed in the filter member 11 had an interval of 10 mm. The edge of the filter 10 on the side opposite from the folded part 11a and one edge of the defoamer 20 were immersed in a polyurethane adhesive that had been poured into a predetermined shaped mold, under which conditions the polyurethane adhesive was hardened so as to form the resin plate 60. The inner vertical dimension Hf of the filter 10 was set to 78 mm. For the defoamer 20, an open-cell polyurethane foam with the surface thereof coated with silicone oil was used. The thickness Tu of the defoamer 20 was set to 10 mm and the vertical dimension Hu thereof was set to 40 mm (see FIGS. 8A and 8B). Two through holes were formed in the resin plate 60. A cylindrical conduit tube 90 having an outer diameter of 10 mm was inserted in one of the two through holes, and a water column manometer was connected to the other through hole so as to measure the pressure in the cardiotomy section.

The aspect ratio ((Ru2-2×Tu)/(Ru1-2×Tu)) of the region surrounded by the lower end of the defoamer 20 was varied by changing the outer dimension Ru2 (see FIG. 8B) of the lower end face of the defoamer 20 in the minor axial direction. In addition, the length Lt of insertion of the conduit tube 90 in the cardiotomy section 2 (see FIG. 8A) was varied.

### [Test Method]

Air-containing (or air bubble-containing) blood (fresh citrated bovine blood, at a temperature of 25°*C*) was caused to flow into the cardiotomy section through the conduit tube 90. The flow rate of blood was varied in six cases, specifically 0.1, 0.2, 0.5, 1.0, 1.5, and 2.0 (×⁻³m³/min), and at each different flow rate of blood, the volume of air contained in blood was varied in four cases, specifically, 0, 0.1, 0.2, and 0.5 (×⁻³m³/min).

### [Evaluation Method]

### a. Internal pressure increase

A change in pressure in the cardiotomy section during the inflow of blood was measured with the water column manometer. The pressure in the cardiotomy section has correlation with the defoaming properties of the cardiotomy section. If the defoaming properties of the cardiotomy section are poor and air bubbles grow up to the upper space 18 (see FIG. 8A), the pressure in the cardiotomy section increases.

### b. Visual observations on condition of air-bubble generation and defoaming condition

Visual observations were made on the condition of air-bubble generation and the defoaming condition in the cardiotomy section during the inflow of blood.

### c. Evaluation

With all factors considered, including the above-described increase in internal pressure and visual observation results, the defoaming properties were classified into three levels: "○" (Excellent), "△" (Ordinary), and "×" (Poor).

### [Evaluation Result]

The evaluation results are shown in Table 1.

**Table 1**

| Aspect Ratio | Lt/Hu | | | | |
|---|---|---|---|---|---|
| | 0.075 | 0.5 | 1.0 | 1.1 | 1.5 |
| 0 | × | × | ○ | ○ | Δ |
| 0.13 | × | × | ○ | ○ | Δ |
| 0.18 | × | × | ○ | ○ | Δ |
| 0.24 | × | × | ○ | ○ | Δ |
| 0.50 | × | × | × | × | × |

As shown in Table 1, if the aspect ratio of the lower end face of the defoamer 20 was higher than 0.24, the defoaming properties were poor. The defoaming properties were also poor if the ratio Lt/Hu of the length Lt of the conduit tube 90 with respect to the vertical dimension Hu of the defoamer 20 was lower than 1.0.

### Experiment 2

A cardiotomy section 2 having the same structure as that in Experiment 1 was created. In Experiment 2, the aspect ratio of the region surrounded by the lower end of the defoamer 20 was set constant at 0.18 and Lt/Hu was set constant at 1.0, whereas the vertical dimension Hu of the defoamer 20 and the length Lt of insertion of the conduit tube 90 in the cardiotomy section 2 were varied.

A test was conducted using the same method as in Experiment 1, and the same method as in Experiment 1 was used for evaluation. The evaluation results are shown in Table 2.

**Table 2**

| Hu/Hf | Evaluation |
|---|---|
| 0.2 | Δ |
| 0.3 | ○ |
| 0.6 | ○ |
| 0.9 | ○ |
| 0.95 | Δ |

As shown in Table 2, when Hu/Hf is from 0.3 to 0.9 inclusive, good defoaming properties were obtained.

The above-described embodiments and examples merely are intended to clarify the technical contents of the present invention and the present invention should not be interpreted to be limited to those embodiments and examples. Various modifications are possible within the scope of the claims and the spirit of the present invention, and the present invention should be interpreted broadly.

### Industrial Applicability

The present invention is widely applicable to blood reservoirs that are provided in an extracorporeal circulation circuit used in cardiopulmonary surgeries or the like.

## Claims

1. A blood reservoir comprising:
a housing including an intracardiac blood inflow port in an upper portion thereof and a blood outflow port at a lower end thereof;
a cardiotomy section arranged in the housing; and
a conduit tube that communicates with the intracardiac blood inflow port and allows blood to flow out of the intracardiac blood inflow port into the cardiotomy section,
the conduit tube being inserted from above downward in the cardiotomy section,
wherein the cardiotomy section includes a filter and a defoamer arranged inside the filter,
a lower end of the filter is sealed or folded in an approximately straight line or in a curve,
the defoamer is arranged annularly along an inner peripheral surface of the filter, as viewed from above,
a lower end of the conduit tube is located at the same level as or at a lower level than a lower end of the defoamer, with respect to a vertical direction,
in a projection view of a lower end face of the defoamer with respect to a horizontal plane, (Ru2-2×Tu)/(Ru1-2×Tu)≤0.24 is satisfied, where Ru1 is an outer dimension of the annularly arranged defoamer in a major axial direction, Ru2 is an outer dimension thereof in a minor axial direction, and Tu is a thickness of the defoamer.

2. The blood reservoir according to claim 1, wherein L/Hu≤0.1 is satisfied, where Hu is a vertical dimension of the defoamer and L is a vertical distance between the lower end of the conduit tube and the lower end of the defoamer.

3. The blood reservoir according to claim 1 or 2, wherein 0.2≤Hu/Hf≤ 0.95 is satisfied, where Hu is a vertical dimension of the defoamer and Hf is a inner vertical dimension of the filter.

4. The blood reservoir according to claim 1 or 2, wherein 0.3≤Hu/Hf≤0.9 is satisfied, where Hu is a vertical dimension of the defoamer and Hf is an inner vertical dimension of the filter.

5. The blood reservoir according to claim 1, wherein a slit that extends upward from the lower end of the conduit tube is formed in a side of the conduit tube, or a through hole is formed at a position on a side of the conduit tube and in the vicinity of the lower end thereof.

6. The blood reservoir according to claim 5, wherein a length Hₛ from the lower end of the conduit tube to an upper end of the slit is from 5 mm to 30 mm inclusive.

7. The blood reservoir according to claim 5, wherein the slit has a width W_{S} of 1 mm to 5 mm inclusive.
